# EUROPEAN PATENT SPECIFICATION

(11) **EP 2 512 806 B1**
(45) Date of publication and mention of the grant of the patent: **04.01.2017**
(21) Application number: 10837133.7
(22) Date of filing: 09.11.2010
(51) Int. Cl.: B32B 39/00, B32B 38/14, B32B 7/06, A61F 13/49, A61L 15/56

(54) **MANUFACTURE AND ASSEMBLY OF ABSORBENT ARTICLES WITH MULTIPLE PRINTED COMPONENTS**
HERSTELLUNG UND ZUSAMMENSETZUNG SAUGFÄHIGER ARTIKEL MIT MEHREREN BEDRUCKTEN KOMPONENTEN
FABRICATION ET ASSEMBLAGE D'ARTICLES ABSORBANTS AUX COMPOSANTS IMPRIMÉS MULTIPLES

(30) Priority: 18.12.2009 US 642226
(43) Date of publication of application: 24.10.2012
(73) Proprietor: Kimberly-Clark Worldwide, Inc., Neenah, Wisconsin 54956 (US)
(72) Inventor: RUMAN, Marcille, Faye, Oshkosh Wisconsin 54901 (US)
(74) Representative: Davies, Christopher Robert
(86) International application number: PCT/IB2010/055099
(87) International publication number: WO 2011/073810

(56) References cited:
- EP-A1- 1 765 252
- EP-A2- 1 455 706
- WO-A2-2011/045684
- US-A1- 2003 088 227
- US-A1- 2004 122 401
- US-A1- 2005 067 083
- US-A1- 2006 047 258
- US-B2- 6 482 192

## Description

### BACKGROUND

The present disclosure relates to producing one or more printed components in a composite absorbent article, and to reducing manufacturing waste in producing the composite article.

A vast number of applications exist in which it is necessary or desirable to monitor the presence and/or position of one or more components of a composite article during manufacturing. For instance, in a largely automated process for manufacturing disposable absorbent articles such as diapers and other incontinence articles, it has long been the industry standard that certain components (e.g., support layers, absorbent pads, elastic components, fastener components, etc.) must be positioned or aligned with respect to each other and/or other components to produce an acceptable article.

Offline flexographic printing can be used to print graphics on side panel materials, but it does not work well in printing registered leg bands. Printed side panel materials can need to be cut, rotated, and placed to align with outercover and back panel die cut out. Registration systems, however, that are necessary to address the printing variability and properly position the leg shape and maintain leg size are inadequate.

### SUMMARY

The present disclosure provides for processes and articles that greatly reduce the amount of waste in the manufacture of disposable absorbent articles having multiple printed components.

Solutions to these problems are presented in a method for manufacturing a disposable absorbent article having multiple printed components, the method including providing a web of side panel having a longitudinal direction, separating a strip from the web of side panel material, wherein the strip has a long dimension, and printing the strip or the web of side panel material with a front leg band stripe, a back leg band stripe, and a waistband stripe prior to attaching the strip to the web of chassis material, wherein the strip has a front leg band stripe portion, a back leg band stripe portion, and a waistband stripe portion. The method also includes attaching the strip to a web of chassis material to form an article assembly web, the web of chassis material having a machine direction (MD), and cutting the article assembly web to produce an individual article.

In addition, a method for manufacturing a disposable absorbent article having multiple printed components includes printing a web of side panel material with a front leg band stripe, a back leg band stripe, and a waistband stripe, the web of side panel material having a longitudinal direction, and printing a web of chassis material with a back leg band segment, the web of chassis material having a machine direction (MD), outer cover material, liner material generally parallel to the outer cover material, and absorbent core material disposed therebetween, wherein the longitudinal direction is substantially parallel to the MD. The method also includes separating a strip from the web of side panel material, the strip having a long dimension and a front leg band stripe portion, a back leg band stripe portion, and a waistband stripe portion, and rotating the strip such that the long dimension is substantially parallel to the MD. The method also includes attaching the strip to the web of chassis material to form an article assembly web, and cutting the article assembly web to produce an individual article.

Other features of the disclosure will be in part apparent and in part pointed out hereinafter. Other objects and advantages of the present disclosure will become more apparent to those skilled in the art in view of the following description and the accompanying drawings.

### BRIEF DESCRIPTION OF THE DRAWINGS

The present disclosure will be more fully understood, and further features will become apparent, when reference is made to the following detailed description and the accompanying drawings. The drawings are merely representative and are not intended to limit the scope of the claims.
Fig. 1 is a side elevation of a child's pants with a fastening system of the pants shown connected on one side of the pants and disconnected on the other side of the pants;
Fig. 2 is a bottom plan view of the pants of Fig. 1 in an unfastened, stretched and laid flat condition to show the surface of the pants that faces away from the wearer;
Fig. 3 is a top plan view of the pants in its unfastened, stretched and laid flat condition to show the surface of the pants that faces the wearer when the pants are worn, with portions of the pants being cut away to reveal underlying features;
Fig. 4 is a schematic view of the pants of Fig. 1 showing a waistband and leg bands;
Fig. 5 is a schematic view of a roll of side panel material used in manufacturing the pants of Fig. 1; and
Fig. 6 is a schematic view of the process equipment used in the manufacture of the pants of Fig. 1.

Repeat use of reference characters in the present specification and drawings is intended to represent the same or analogous features or elements of the present disclosure. The drawings are representational and are not necessarily drawn to scale. Certain proportions thereof might be exaggerated, while others might be minimized.

### DETAILED DESCRIPTION

It is to be understood by one of ordinary skill in the art that the present discussion is a description of exemplary aspects of the present disclosure only, and is not intended as limiting the broader aspects of the present disclosure.

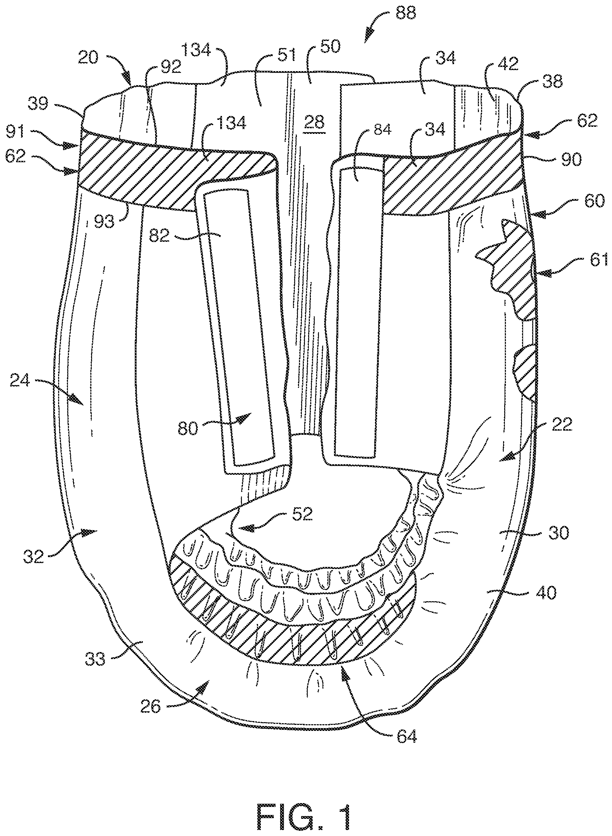

The methods and apparatus of the present disclosure can be used to make a variety of pre-fastened articles such as disposable absorbent garments including diapers, training pants, feminine hygiene products, incontinence products, medical garments, other personal care or health care garments, swim pants, athletic clothing, pants and shorts, and the like. More particularly, the methods and apparatus of the present disclosure can be used to make articles in which at least two elements of the article are connected together during the making thereof to assemble or "pre-fasten" the article. For ease of explanation, the methods and apparatus of the present disclosure are hereafter described in connection with making pre-fastened child's pants, generally indicated as 20 in Fig. 1. In particular, the methods and apparatus will be described in terms of those for making pre-fastened disposable pants as described in U.S. Patent Application Serial Number 09/444,083 titled "Absorbent Articles With Refastenable Side Seams" and filed November 22, 1999 (corresponding to PCT application WO 00/37009 published June 29, 2000) by A. L. Fletcher et al., the disclosure of which is incorporated herein by reference. Absorbent article 20 can also be constructed using the methods and apparatus disclosed in U.S. Patent 4,940,464 issued July 10,1990 to Van Gompel et al.; and U.S. Patent 5,766,389 issued June 16, 1998 to Brandon et al.; the disclosures of which are also incorporated herein by reference.

It should be understood that as used herein, the term "component" includes not only discrete objects, but also objects yet to be formed into discrete objects (e.g., objects yet to be severed into discrete objects from a continuous sheet or web of material), particles (e.g., superabsorbent particles or polymers), adhesives, lotions, ointments, and other substances, as well as portions or characteristics of any such components including, for example, fold lines, bond lines (e.g., ultrasonic bond lines), bonded or adhered regions, and registration marks applied to or about components for subsequent detection during a manufacturing or inspection process.

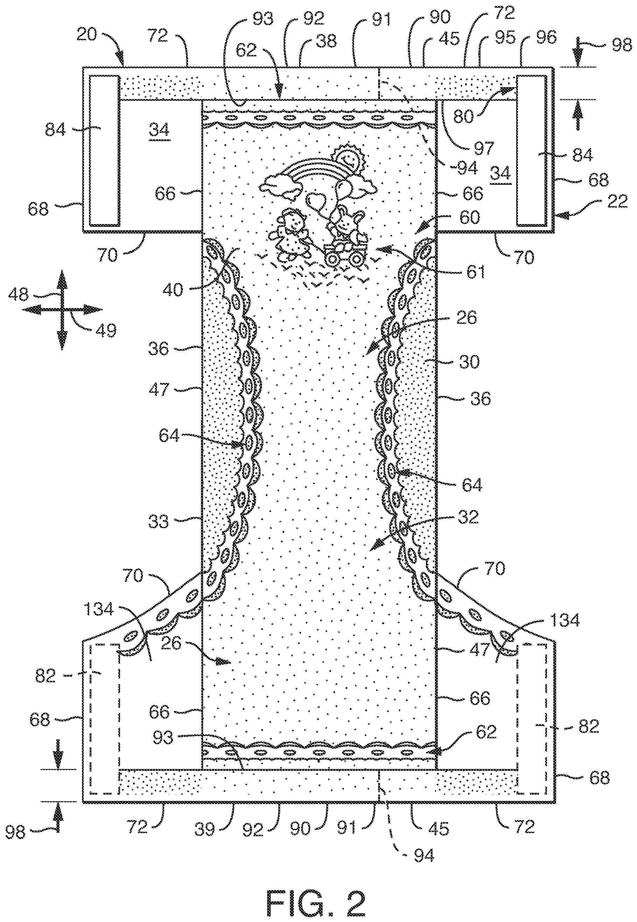

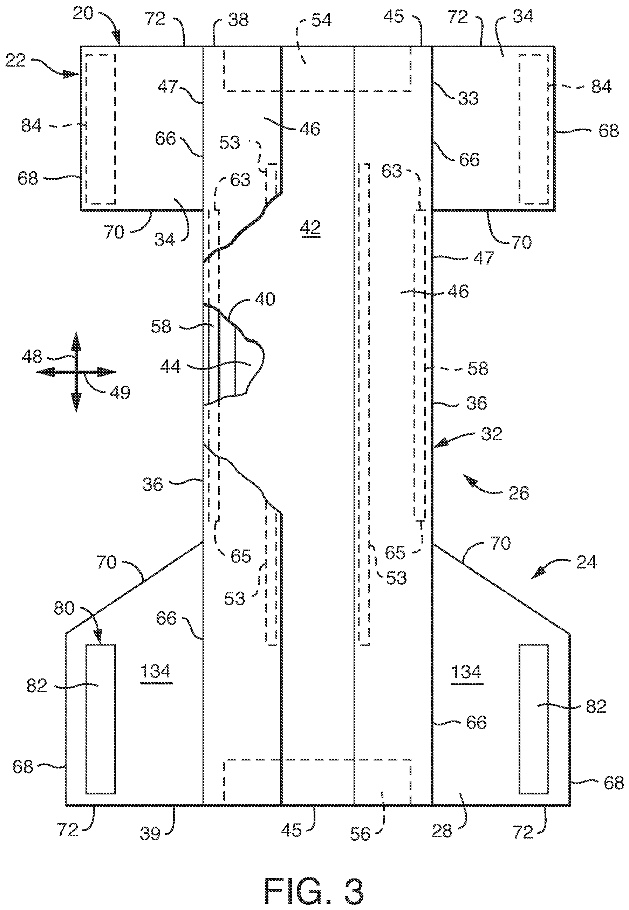

With reference now to the drawings, and in particular to Fig. 1, absorbent article 20 is illustrated in a partially fastened condition and include an absorbent chassis 32 having a front waist region 22, a back waist region 24, a crotch region 26 interconnecting the front and back waist regions 22, 24, an inner surface 28 that is configured to contact the wearer, and an outer surface 30 opposite the inner surface and configured to contact the wearer's clothing. With additional reference to Figs. 2 and 3, the absorbent chassis 32 also has a pair of laterally opposite side edges 36 and a pair of longitudinally opposite waist edges, respectively designated front waist edge 38 and back waist edge 39. The front waist region 22 is contiguous with the front waist edge 38, and the back waist region 24 is contiguous with the back waist edge 39.

The illustrated absorbent chassis 32 includes a composite structure 33, which when laid flat can be rectangular or any other desired shape, and has a pair of laterally opposite front side panels 34 and a pair of laterally opposite back side panels 134 extending outward therefrom.

The composite structure 33 and side panels 34, 134 can include two or more separate elements, as shown in Fig. 1, or be integrally formed. Integrally formed side panels 34, 134 and composite structure 33 would include at least some common materials, such as the bodyside liner, flap composite, outer cover, other materials and/or combinations thereof, and could define a one-piece elastic, stretchable, or non-stretchable pants. The illustrated composite structure 33 includes an outer cover 40, a bodyside liner 42 (Figs. 1 and 3) connected to the outer cover in a superposed relation, an absorbent assembly 44 (Fig. 3) disposed between the outer cover and the bodyside liner, and a pair of containment flaps 46 (Fig. 3). The illustrated composite structure 33 has opposite ends 45 (Figs. 2 and 3) that form portions of the front and back waist edges 38 and 39, and opposite side edges 47 that form portions of the side edges 36 of the absorbent chassis 32 (Figs. 2 and 3).

For reference, arrows 48 and 49 (Figs. 2 and 3) depict the orientation of the longitudinal axis and the transverse or lateral axis, respectively, of the absorbent article 20.

With the absorbent article 20 in the fastened position as partially illustrated in Fig. 1, the front and back side panels 34, 134 are connected together by a fastening system 80 to define a three-dimensional pants configuration having an interior space 51, a waist opening 50 for receiving the wearer into the interior space of the pants, a pair of leg openings 52 and engagement seams 88 along which the side panels 34, 134 are connected. The interior space 51 of the absorbent article 20 is thus bounded by the absorbent chassis 32, the engagement seams 88 and the portions of the side panels 34, 134 extending on opposite sides of the engagement seams 88 (e.g., between the engagement seams 88 and the absorbent chassis 32). As used herein, the "interior space" 51 is intended to refer to the space between any two portions of a three-dimensional article that generally oppose each other. It is understood that a transverse cross-section of the article need not be closed, e.g., continuous, to define the interior space 51. For example, a two-dimensional article can be generally folded over on itself so that two portions of the article oppose each other to define an interior space of the article therebetween. Thus, the interior space 51 of the absorbent article 20 shown in Fig. 1 can be defined by the side panels 34, 134 themselves or, if the side panels are fully straightened therebetween, the interior space is defined by a combination of the side panels and the front and back waist regions 22, 24 of the absorbent chassis 32.

The front waist region 22 includes the portion of the absorbent article 20 which, when worn, is positioned on the front of the wearer while the back waist region 24 includes the portion of the pants which, when worn, is positioned on the back of the wearer. The crotch region 26 of the absorbent article 20 includes the portion of the absorbent article 20 which, when worn, is positioned between the legs of the wearer and covers the lower torso of the wearer. The front and back side panels 34 and 134 include the portions of the absorbent article 20 which, when worn, are positioned on the hips of the wearer. The waist edges 38 and 39 of the absorbent chassis 32 are configured to encircle the waist of the wearer when worn and together define the waist opening 50 (Fig. 1). Portions of the side edges 36 in the crotch region 26 generally define the leg openings 52.

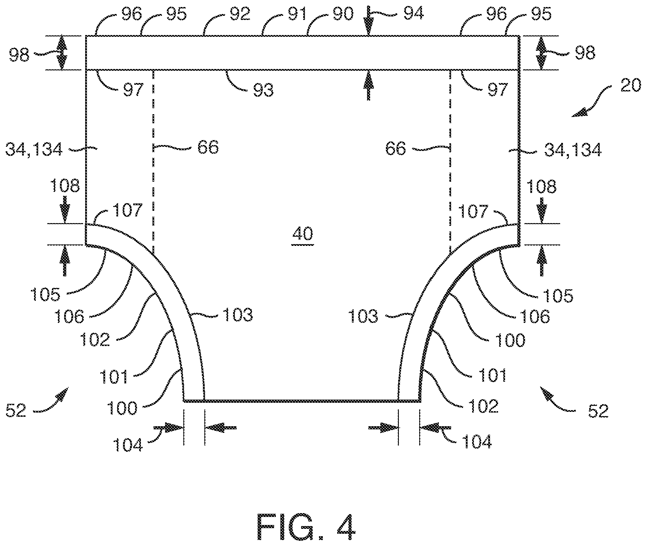

In another aspect of the present disclosure best illustrated in Figs. 2 and 4, the absorbent article 20 also include a printed waistband 90. The printed waistband 90 includes a waistband outer cover portion 91 disposed on the absorbent chassis 32 adjacent the waist opening 50. The waistband outer cover portion 91 includes a distal edge 92, a proximal edge 93, and a longitudinal height 94, which is the longitudinal distance between the proximal edge 93 and the distal edge 92. The printed waistband 90 also includes a waistband first side panel portion 95 disposed on one of the side panels 34, 134 adjacent the waist opening 50. The waistband first side panel portion 95 includes a distal edge 96, a proximal edge 97, and a longitudinal height 98, which is the longitudinal distance between the proximal edge 97 and the distal edge 96. In addition, each other article component adjacent the waist opening 50 can have a portion of the printed waistband 90 with similar features.

In practice, the distal edges 92, 96 of the waistband outer cover portion 91 and of the waistband first side panel portion 95 can be longitudinally aligned or can be longitudinally offset. The proximal edges 93, 97 of the waistband outer cover portion 91 and of the waistband first side panel portion 95 can be longitudinally aligned or can be longitudinally offset. Offsets, misalignment, and misalignment offsets refer to the longitudinal positioning of an edge of a component or a feature as compare to the longitudinal positioning of an edge of another component or feature.

In addition, the longitudinal heights 94, 98 of the waistband outer cover portion 91 and of the waistband first side panel portion 95 can be equal or unequal. Further, the waistband outer cover portion 91 and the waistband first side panel portion 95 can have no lateral gap therebetween in the lateral direction, or can be disposed such that a lateral gap is present between the portions.

In another aspect of the present disclosure best illustrated in Fig. 4, the absorbent article 20 also include a printed leg band 100. The printed leg band 100 includes a leg band outer cover portion 101 disposed on the absorbent chassis 32 adjacent the leg opening 52. The leg band outer cover portion 101 includes a distal edge 102, a proximal edge 103, and a longitudinal height 104, which is the longitudinal distance between the proximal edge 103 and the distal edge 102. The printed leg band 100 also includes a leg band first side panel portion 105 disposed on one of the side panels 34, 134 adjacent the leg opening 52. The leg band first side panel portion 105 includes a distal edge 106, a proximal edge 107, and a longitudinal height 108, which is the longitudinal distance between the proximal edge 107 and the distal edge 106. In addition, each other article component adjacent the leg opening 52 can have a portion of the printed leg band 100 with similar features. The absorbent article 20 preferably includes a printed leg band 100 encircling each leg opening 52.

As used herein, the longitudinal height of a printed waistband 90 or of a printed leg band 100 is generally the distance between the distal and proximal edges of that band. Some bands, however, do not have linear edges. For example, bands can be scalloped, sinusoidal, arcuate, or of any other non-linear design. In such cases, the longitudinal height is the greatest distance between a distal edge and a proximal edge.

In practice, the distal edges 102, 106 of the leg band outer cover portion 101 and of the leg band first side panel portion 105 can be longitudinally aligned or can be longitudinally offset. The proximal edges 103, 107 of the leg band outer cover portion 101 and of the leg band first side panel portion 105 can be longitudinally aligned or can be longitudinally offset. In addition, the longitudinal heights 104, 108 of the leg band outer cover portion 101 and of the leg band first side panel portion 105 can be equal or unequal. Further, the leg band outer cover portion 101 and the leg band first side panel portion 105 can have no lateral gap therebetween in the lateral direction, or can be disposed such that a lateral gap is present between the portions.

The absorbent chassis 32 is configured to contain and/or absorb any exudates discharged from the wearer. For example, the absorbent chassis 32 desirably, although not necessarily, includes the pair of containment flaps 46 that are configured to provide a barrier to the transverse flow of body exudates. A flap elastic member 53 (Fig. 3) can be operatively joined with each containment flap 46 in any suitable manner as is well known in the art. The elasticized containment flaps 46 define an unattached edge that assumes an upright configuration in at least the crotch region 26 of the absorbent article 20 to form a seal against the wearer's body. The containment flaps 46 can be located along the side edges 36 of the absorbent chassis 32, and can extend longitudinally along the entire length of the absorbent chassis 32, or can only extend partially along the length of the absorbent chassis 32. Suitable constructions and arrangements for the containment flaps 46 are generally well known to those skilled in the art and are described in U.S. Patent 4,704,116 issued November 3, 1987 to Enloe, which is incorporated herein by reference.

To further enhance containment and/or absorption of body exudates, the absorbent article 20 desirably, although not necessarily, include a front waist elastic member 54, a rear waist elastic member 56, and leg elastic members 58, as are known to those skilled in the art (Fig. 3). The waist elastic members 54 and 56 can be operatively joined to the outer cover 40 and/or the bodyside liner 42 along the opposite waist edges 38 and 39, and can extend over part or all of the waist edges. The leg elastic members 58 can be operatively joined to the outer cover 40 and/or the bodyside liner 42 along the opposite side edges 36 and positioned in the crotch region 26 of the absorbent article 20. The leg elastic members 58 can be longitudinally aligned along each side edge 47 of the composite structure 33. Each leg elastic member 58 has a front terminal point 63 and a back terminal point 65, which represent the longitudinal ends of the elastic gathering caused by the leg elastic members. The front terminal points 63 can be located adjacent the longitudinally innermost parts of the front side panels 34, and the back terminal points 65 can be located adjacent the longitudinally innermost parts of the back side panels 134.

As shown in Figs. 1 and 2, the absorbent article 20 and in particular the outer cover 40 desirably includes one or more appearance-related components. Examples of appearance-related components include, but are not limited to, graphics; highlighting or emphasizing leg and waist openings in order to make product shaping more evident or visible to the user; highlighting or emphasizing areas of the product to simulate functional components such as elastic leg bands, elastic waistbands, simulated "fly openings" for boys, ruffles for girls; highlighting areas of the product to change the appearance of the size of the product; registering wetness indicators, temperature indicators, and the like in the product; registering a back label, or a front label, in the product; and registering written instructions at a desired location in the product.

The illustrated absorbent article 20 is designed for use by young girls and includes a registered outer cover graphic 60 (Figs. 1 and 2). In this design, the registered graphic 60 includes a primary pictorial image 61, simulated waist ruffles 62, and simulated leg ruffles 64. The primary pictorial image 61 includes an object graphic such as a rainbow, sun, clouds, animal characters, wagon and balloons. Any suitable design can be utilized for pants intended for use by young girls, so as to be aesthetically and/or functionally pleasing to them and the caregiver. The appearance-related components are desirably positioned on the absorbent article 20 at selected locations, which can be carried out using the methods disclosed in U.S. Patent No. 5,766,389 issued June 16, 1998 to Brandon et al., the entire disclosure of which is incorporated herein by reference. The primary pictorial image 61 is desirably positioned in the front waist region 22 along the longitudinal center line of the absorbent article 20.

The printed graphics on the absorbent article 20 can share a color palette, can be of the same color, can be of complementary colors, or can follow any suitable color scheme. Different areas of the absorbent article 20 can also be printed with similar or identical graphical elements such as stars, circles, butterflies, etc. Different areas of the absorbent article 20 can also be printed with similar or identical graphics that share a graphic theme. For example, the absorbent article 20 can be printed with flowers that might or might not be identical in size, type, color, etc., but that all share the flower theme. In another aspect, the article can be reverse printed such that graphical elements, for example, appear as an absence of printing.

As noted previously, the illustrated absorbent article 20 has front and back side panels 34 and 134 disposed on each side of the absorbent chassis 32. The front side panels 34 can be permanently bonded along seams 66 to the composite structure 33 of the absorbent chassis 32 in the respective front and back waist regions 22 and 24. More particularly, as seen best in Figs. 2 and 3, the front side panels 34 can be permanently bonded to and extend transversely outward beyond the side edges 47 of the composite structure 33 in the front waist region 22, and the back side panels 134 can be permanently bonded to and extend transversely outward beyond the side edges of the composite structure in the back waist region 24. The side panels 34 and 134 can be bonded to the composite structure 33 using attachment means known to those skilled in the art such as adhesive, thermal or ultrasonic bonding. Alternatively, the side panels 34 and 134 can be formed as an integral portion of a component of the composite structure 33. For example, the side panels can include a generally wider portion of the outer cover 40, the bodyside liner 42, and/or another component of the absorbent chassis 32. The front and back side panels 34 and 134 can be permanently bonded together or be releasably connected with one another such as by the fastening system 80 of the illustrated aspect.

As best illustrated in Figs. 2 and 3, the front and back side panels 34, 134 each have an outer edge 68 spaced laterally from the seam 66, a leg end edge 70 disposed toward the longitudinal center of the absorbent article 20, and a waist end edge 72 disposed toward a longitudinal end of the pants. The leg end edge 70 and waist end edge 72 extend from the side edges 47 of the composite structure 33 to the outer edges 68. The leg end edges 70 of the side panels 34 and 134 form part of the side edges 36 of the absorbent chassis 32. In the back waist region 24, the leg end edges 70 are desirably, although not necessarily, curved and/or angled relative to the transverse axis 49 to provide greater coverage toward the back of the absorbent article 20 as compared to the front of the pants. The waist end edges 72 are desirably parallel to the transverse axis 49. The waist end edges 72 of the front side panels 34 form part of the front waist edge 38 of the absorbent chassis 32, and the waist end edges 72 of the back side panels 134 form part of the back waist edge 39 of the absorbent chassis. The waist end edges 72 are generally aligned or co-linear with the front and back waist edges 38, 39.

In particular aspects for improved fit and appearance, the side panels 34, 134 desirably have an average length measured parallel to the longitudinal axis 48 that is about 15 percent or greater, and particularly about 25 percent or greater, of the overall length of the pants, also measured parallel to the longitudinal axis 48. For example, in an absorbent article 20 having an overall length of about 54 centimeters, the side panels 34, 134 desirably have an average length of about 10 centimeters or greater, such as about 15 centimeters. While each of the side panels 34, 134 extends from the waist opening 50 to one of the leg openings 52, the illustrated back side panels 134 have a continually decreasing length dimension moving from the seam 66 to the outer edge 68, as is best shown in Figs. 2 and 3.

Each of the side panels 34, 134 can include one or more individual, distinct pieces of material. In particular aspects, for example, each side panel 34, 134 can include first and second side panel portions that are joined at a seam, or can include a single piece of material that is folded over upon itself (not shown).

The side panels 34, 134 desirably, although not necessarily, include an elastic material capable of stretching in a direction generally parallel to the transverse axis 49 of the absorbent article 20. Suitable elastic materials, as well as one process of incorporating elastic side panels into pants, are described in the following U.S. Patents: 4,940,464 issued July 10, 1990 to Van Gompel et al.; 5,224,405 issued July 6, 1993 to Pohjola; 5,104,116 issued April 14, 1992 to Pohjola; and 5,046,272 issued September 10, 1991 to Vogt et al.; all of which are incorporated herein by reference. An alternative elastic material is described below. In particular aspects, the elastic material includes a stretch-thermal laminate (STL), a neck-bonded laminate (NBL), a reversibly necked laminate, or a stretch-bonded laminate (SBL) material. Methods of making such materials are well known to those skilled in the art and described in U.S. Patent 4,663,220 issued May 5, 1987 to Wisneski et al.; U.S. Patent 5,226,992 issued July 13, 1993 to Morman; and European Patent Application No. EP 0 217 032 published on April 8, 1987 in the names of Taylor et al.; all of which are incorporated herein by reference. Alternatively, the side panel material can include other woven or nonwoven materials, such as those described above as being suitable for the outer cover 40 or bodyside liner 42; mechanically pre-strained composites; or stretchable but inelastic materials.

Further detail with respect to elastic laminates of the present disclosure can be found in co-pending U.S. Patent Publication No. 2008/0095978 entitled "Nonwoven Composite Containing An Apertured Elastic Film," which is incorporated herein by reference to the extent it does not conflict herewith.

Absorbent article 20 can have the side panels 34, 134 affixed to each other for securing the absorbent article 20 about the waist of the wearer. The side panels 34, 134 can be affixed by bonding, mechanical fasteners, or any other suitable method, and can be affixed permanently, in a tearable manner, or in a refastenable manner. The illustrated absorbent article 20 includes the fastening system 80 for refastenably securing the pants about the waist of the wearer. The illustrated fastening system 80 includes first fastening components 82 adapted for refastenable engagement to corresponding second fastening components 84. In one aspect, one surface of each of the first fastening components 82, 84 includes a plurality of engaging elements that project from that surface. The engaging elements of the first fastening components 82 are adapted to repeatedly engage and disengage engaging elements of the second fastening components 84.

The fastening components 82, 84 can include separate elements bonded to the side panels 134, 34, or they can be integrally formed with the side panels. Thus, unless otherwise specified, the term "fastening component" includes separate components that function as fasteners, and regions of materials such as the side panels 34, 134 that function as fasteners. Moreover, a single material can define multiple fastening components to the extent that different regions of the material function as separate fasteners. The fastening components 82, 84 can be located on the side panels 134, 34, between the side panels such as on the absorbent chassis, or a combination of the two.

Components, alignment, and articles can be inspected using an infrared inspection system such as that described in U.S. 6,885,451 to Vogt, et al., and in co-pending U.S. Patent Application Serial No. 12/580,765, entitled "Disposable Absorbent Articles Incorporating Black-Free Colorant" each of which is incorporated herein by reference to the extent it does not conflict herewith.

As described above, waist and leg openings of disposable absorbent articles are often created by joining multiple components. The present disclosure applies in particular to articles for which the circumference of either the waist or leg opening has more than two interfaces or junctions at which components are attached, bonded, etc., as is described in more detail above. The article can include one or more printed band features in the form of a waistband partially or completely encircling the waist opening and/or a leg band partially or completely encircling one or each leg opening. For example, a fully encircling waistband is achieved by attaching a pair of right and left side panels to each of the front and rear waist regions of an article's outercover and then bonding the front and back assemblies together at the sides to create a closed garment. In this example, the waistband encompasses six components (with the outer cover counting twice with its front and rear waist regions) and six junctions. In other aspects, a functional waistband that does or does not span the entire waist circumference can be attached to the outercover and/or side panels with similar complexity. The printed band feature can be of uniform or any other suitable appearance. Multiple printed band features, such as a waistband including a plurality of generally horizontal and adjoining lines, are considered to be a single band feature

Similarly, a fully encircling leg band is also achieved by attaching a pair of right and left side panels to each of the front and rear waist regions of an article's outercover and then bonding the front and back assemblies together at the sides to create a closed garment. In this example, each leg band encompasses three components and three junctions. Aligning each of these components properly is a complex process that is further complicated if printing on some or all components also needs to be aligned. In other aspects, functional leg bands and leg elastics that do not span the entire leg circumference can be attached to the outercover and/or side panels.

Printing fully-encircling waist and leg bands provides a visual cue to a consumer of a fully-encircling functional waistband, which is a key component in giving the article an appearance of conventional underwear. Printing is leveraged to create the visual perception of a continuous waist and/or leg band material that exists across multiple pieces (e.g., side panels, outercover, waist elastics, and leg elastics). The printing can be applied to the garment or outward facing surfaces of the outer cover and side panels, the body or inward facing surfaces of the outercover and side panels, and/or sandwiched within material layers.

In an alternative aspect of the present disclosure, a waistband and/or a leg band is not required to have a distinct or abrupt edge. A gradation in the printing, such as a gradation from darker to lighter, can be employed for visual effect and, in some cases, to minimize the appearance of a misalignment offset. The same acceptable/unacceptable offset limits and features described herein can be applied to such a gradation with respect to its edges and/or longitudinal height.

The components can be printed prior to, during, or after the multiple components are combined to form the absorbent article. The printed waist/leg bands are substantially but not necessarily completely aligned at the seams (e.g., outercover to side panel, front to back side seams) and substantially encircle the waist and leg openings. The article can have additional printing or pigmentation to further enhance a more uniform, garment-like look.

Another aspect of the present disclosure recognizes the insensitivity of the primary user of the articles to misalignment or offset as described above. In this aspect, an offset is set at which the article is still acceptable and thus not subject to culling and being discarded. For example, the distal edges of the waistband outer cover portion and of the waistband first side panel portion can be longitudinally offset, or offset by differing in longitudinal height, depending on underwear style, size, intended user age, and gender emulated by the disposable articles. Additional information with respect to offsets and misalignments of waist and leg bands can be found in co-pending U.S. Patent Application Serial No. 12/580,529 to Ruman, et al. and entitled "Alignment of Leg and Waist Bands on Disposable Absorbent Articles," which is incorporated herein to the extent it does not conflict herewith.

Additionally, an article constructed from multiple components can have a fully-encircling printed waistband printed on both the inside and outside of the article to mimic a functional waistband. The article can also have additional functional waistband materials and components that overlap the side panels. In this latter case, a printed waistband can also be added, and can be partially or completely printed on all or a portion of the functional waistband.

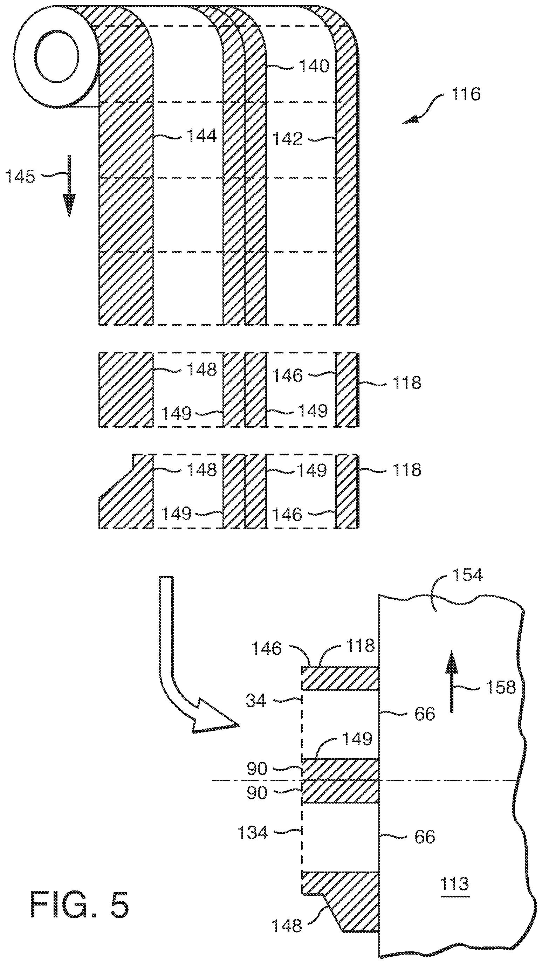

To address the inherent problems associated with pre-printed, registered leg band graphics, an alternative means to create leg bands has been identified. Three stripes are printed on a roll of side panel material 116, as illustrated in Fig. 5. The stripes run generally in the MD, and are spaced apart in the CD. The stripes are not registered in the MD. The side panel material 116 can be printed on any surface of any layer of the side panel material 116. In the process of manufacturing absorbent article 20, a middle stripe or waistband stripe 140 will form both the front and back waistbands 90, a first edge stripe or front leg band stripe 142 will form the front leg band 146, and the third edge stripe or back leg band edge stripe 144 will form the back leg band 148. Any part of the side panel material 116 can also be printed with other graphics related or unrelated to the stripes and/or the outer cover graphic 60.

Any or each of the first edge, middle, and third edge stripes 142, 140, 144 can be patterned, solid, scalloped, linear, non-linear, multiple stripes, or any other suitable design. Certain designs, such as a scalloped design, can be used to help mask any potential misalignment between the side panels 34, 134 and the chassis 33. In alternate aspects of the present disclosure, the design of the first and third edge stripes 142, 144 do not need to replicate each other or the middle stripe 140 in design, color, etc. In other aspects, the first and/or third edge stripes 142, 144 can be printed to match elements of the outer cover graphic 60. For example, a spray-like pattern of repetitive dots can appear in both the outer cover graphic 60 and in or as one or both of the first and third edge stripes 142, 144. Any stripe, and particularly the middle stripe 140, can be printed to include an unprinted stripe therein, so as to appear in one example as two parallel lines or stripes.

The third edge stripe 144 that becomes the back leg band 148 can appear significantly wider than the front leg band stripe 142 because a majority of the back leg band material will be removed when the back leg band region is die cut to the appropriate leg opening shape. In other aspects of the present disclosure, the third edge stripe 144 that becomes the back leg band 148 can also be printed in a non-linear manner such as a repeated arcuate pattern to avoid printing material that will be removed by die-cutting, or to optimize the shape of the back leg band 148. The third edge stripe 144 that becomes the back leg band 148 can also be printed in a discontinuous manner, including printing in a repetitive chevron or check mark-shaped pattern or printing repetitive triangles of any suitable design. The third edge stripe 144 can be printed such that a part or all of the third edge stripe 144 is at an angle to the middle stripe 140, or that a part or all of the third edge stripe 144 is parallel to the middle stripe 140. The third edge stripe 144 can be printed to match the pending side panel die cut to minimize printing material that will be discarded.

Because the outer cover 40 can have printed leg band graphics that are curved and/or shaped, the leg band thickness or longitudinal height 108 can be modified to align with the side panel leg bands 146, 148 at the outer cover/side panel seams 66 in the front and back of the pant.

A method for cutting and placing a side panel on a training pant is described in U.S. Patent No. 6,513,221 to Vogt et al. and entitled "Garment Side Panel Conveyor System and Method," which is incorporated herein to the extent it does not conflict herewith. In addition, one suitable applicator device is disclosed in more detail in U.S. Pat. No. 5,104,116 issued Apr. 14, 1992 and U.S. Pat. No. 5,224,405 issued Jul. 6, 1993 both to Pohjola, which are incorporated herein to the extent they do not conflict herewith.

Continuous webs of side panel material 116 used to form the side panels 34 and 134 can be provided from suitable supply sources. The supply sources can include one or more standard unwind mechanisms. The side panel material 116 is printed with graphics that are not registered in the longitudinal direction of the web of side panel material 116. The side panel material graphics can include a waistband stripe, a front leg band stripe, and a back leg band stripe, curve, chevron, or other suitable back leg band graphic. Because of the arrangement of stripes, each roll of printed side panel material 116 is specific to a side. The side panel material 116 can include all or some of the materials and layers of the side panels 34, 134 of the absorbent article 20. For example, the side panel material 116 can be applied to the absorbent article 20 such that the side panels 34, 134 have the same materials and layers as the side panel material 116, or the side panel material 116 can be applied to the absorbent article 20 such that the side panels 34, 134 are only formed with the addition of other material(s), such as the outer cover 40.

In one aspect of the present disclosure, and the aspect discussed primarily herein, the side panel material 116 has MD stretch as it is disposed on the roll, and rotation of strips 118 of side panel material 116 as described below allows CD stretch of the side panel material 116 as it is disposed in the absorbent article 20. In other aspects of the present disclosure, the side panel material 116 has CD stretch as it is disposed on the roll, and rotation of strips 118 of side panel material 116, as described below, is not required to allow CD stretch of the side panel material 116 as it is disposed in the absorbent article 20.

In one aspect of the manufacturing process, the roll of side panel material 116 is arranged such that the longitudinal direction 145 of the web of side panel material 116 is substantially parallel to the MD 158. In other aspects, the longitudinal direction 145 can be in any other arrangement or disposition.

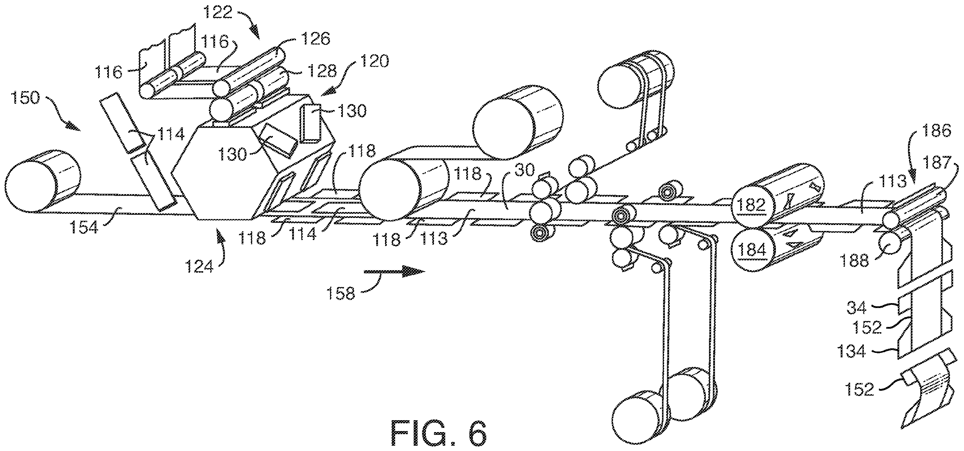

An exemplary embodiment of an assembly section 150 for making a continuous stream of partially assembled, discrete articles 152 is illustrated in Fig. 6. The specific equipment and processes used in the assembly section 150 can vary greatly depending on the specific type of garment being manufactured. The particular process and apparatus described in relation to FIG. 6 is specifically adapted to manufacture training pants 20 of the type illustrated in Figs. 1-4.

Continuous webs of material to form the outer cover 40 are printed with an MD-registered graphic and are also provided from suitable supply sources. Orientation of the outer cover graphics as the outer cover material is taken off of its roll is maintained as the materials are converted into training pants, which is an MD process. The supply sources can include one or more standard unwind mechanisms. The outer cover material and/or other webs of chassis materials can be printed with back leg band segments, front leg band segments, and waistband segments to augment the underwear-like look of the finished training pants.

The side panel material 116 can be cut into individual strips 118 and positioned partially on the chassis material 154 using an applicator device 120. Each strip 118 has a long dimension and portions of the printed stripes: a front leg band stripe portion 146, a back leg band stripe portion 148, and a waistband stripe portion 149. When the web of side panel material 116 is arranged such that the longitudinal direction 145 is substantially parallel to the MD 158, the long dimension of the strip will be substantially perpendicular to the MD 158 upon cutting. In this aspect, and after cutting, the individual strips 118 are rotated as described below and positioned partially on either the outer cover or bodyside liner material (the chassis material 154) using an applicator device 120. Generally speaking, the side panel material 116 is cut into a strip 118, rotated 90 degrees, and placed adjacent to and attached to the chassis material 154. The individual strips 118 desirably extend laterally outward in the cross machine direction from the chassis material 154 and overlap the chassis material 154 by an amount such as about 2 or more centimeters to permit bonding of the strips to the chassis. In the MD 158, the position of the strips 118 can be registered relative to the absorbent assemblies 114 so that the product assemblage 113 can be cut between the absorbent assemblies 114 with each strip 118 of side panel material forming both a front side panel 34 and a back side panel 134 of consecutive articles 152. In another aspect, also in the machine-direction, the position of the strips 118 can be registered to align the back leg band stripe portion with the back leg band segment.

The applicator device 120 can include a cutting assembly 122 and a rotatable transfer roll 124. The cutting assembly 122 employs a rotatable knife roll 126 and a rotatable vacuum anvil roll 128 to cut individual strips 118 from the continuous side panel material 116. The strips 118 cut by a blade on the knife roll 126 can be maintained on the anvil roll 128 by vacuum and transferred to the transfer roll 124.

The rotatable transfer roll 124 can include a plurality of rotatable vacuum pucks 130. The vacuum pucks 130 receive the strips 118 of material 116 from the cutting assembly 122 and rotate and transfer the strips 118 to the continuously moving chassis material 154. This web of chassis material 154 includes outer cover material, liner material generally parallel to the outer cover material, and absorbent core material 114 disposed therebetween. When the strips 118 are positioned as desired relative to the chassis material 154, the strips 118 are released from the pucks 130 by extinguishing the vacuum in the pucks 130. The pucks 130 can continue to rotate toward the cutting assembly 122 to receive other strips 118. Correct placement of the side panel strip 118 relative to the chassis material 154 is important to maintain the appropriate leg size and to align the waist and leg band graphics. The strips 118 are attached to the web of chassis material 154 to form an article assembly web 113.

The material 116 used to form the side panels 34, 134 can alternatively be provided in continuous form and pressurized fluid-jets or a rotary die cutter can be employed to cut the material to form leg openings 52. Still alternatively, the side panels 34 and 134 of the training pant 20 can be provided by portions of the bodyside liner 42 and/or outer cover 40.

The strips 118 of side panel material 116 can be trimmed if desired, for example to provide angled and/or curved leg end edges 70 in the back waist region 24. To this end, the assembly section 150 can include a die cutting roll 182 and a backing roll 184. A portion of each strip 118 is trimmed from the trailing edge to form the angled and/or curved leg end edges 70 in the back waist region 24. Die cutting can be performed such that the die cut is in registry with the back leg band strip portion. Additional cutting of the side panel can be formed after the side panel is attached to the article assembly web 113. Cutting as described herein can be performed using a die cutter or any other suitable cutting method.

The process described herein can include registering the printing on the chassis 33 or outer cover 40, registering the printing on a side panel 34, 134, registering the attachment of the side panel 34, 134 to article assembly web 113, and registering the cuts made to the article assembly web 113.

The method and apparatus to this point provides a continuous web of interconnected and partially assembled absorbent articles moving in the direction indicated by arrow 158. This continuously moving article assembly web 113 is passed through a cutter 186 that selectively cuts the web into discrete, partially assembled absorbent article 152. Cutting the article assembly web 113 can include cutting the strip 118 within the waistband stripe portion 149. Such cutters 186 are generally known to those skilled in the art and can include, for example, the combination of a cutting roll 187 and an anvil roll 188 through which the web travels. The anvil roll 188 can include a hardened steel rotating roll while the cutting roll 187 can include one or more flexible hardened steel blades clamped onto another rotating roll. The pinching force between the blade on the cutting roll 187 and the anvil roll 188 creates the cut. The cutting roll 187 can have one or more blades depending upon the desired distance between the cuts. The cutter 186 can further be configured to provide spacing between the individual cut pieces after they are cut. Such spacing can be provided by transferring the cut pieces away from the cutter at a higher speed than the speed at which the web is provided to the cutter.

The discrete absorbent article 152 can then be folded at a folding station using any suitable folding mechanism. The absorbent article 152 can be folded about a fold line generally bisecting the absorbent article. As such, the waist regions 22 and 24 of each training pant 102 are positioned in facing relationship with the side panels 34 and 134 extending laterally outward relative to the longitudinal axis 48 of the training pant. The fold line extends in a lateral direction through the crotch region 26 of the training pant. Desirably, each discrete training pant 102 is consistently folded about the fold line such that the front and back waist edges 38 and 39 of the training pant align with each other.

While a side panel and outer cover chassis design is disclosed above, the present disclosure can apply equally to conventional diapers, boxer-type disposable articles, pull-on underwear, adjustable underwear, disposable underwear, and belted shields.

When introducing elements of the present disclosure or the preferred aspect(s) thereof, the articles "a", "an", "the", and "the" are intended to mean that there are one or more of the elements. The terms "comprising," "including", and "having" are intended to be inclusive and mean that there can be additional elements other than the listed elements.

The disclosure has been described with reference to various specific and illustrative aspects and techniques. However, it should be understood that many variations and modifications can be made while remaining within the spirit and scope of the disclosure. Many alternatives, modifications and variations will be apparent to those skilled in the art in light of the foregoing description. Accordingly, this disclosure is intended to embrace all such alternatives, modifications, and variations that fall within the spirit and scope of the appended claims.

## Claims

1. A method for manufacturing a disposable absorbent article (20) having multiple printed components, the method comprising:
providing a web of side panel (116) having a longitudinal direction;
separating a strip (118) from the web of side panel material (116), wherein the strip (118) has a long dimension;
wherein the method comprises printing the strip (118) or the web (116) of side panel material with a front leg band stripe (142), a back leg band stripe (144), and a waistband stripe (140) prior to attaching the strip (118) to the web of chassis material (154), wherein the strip (118) has a front leg band stripe portion (146), a back leg band stripe portion (148), and a waistband stripe portion (149);
attaching the strip (118) to a web of chassis material (154) to form an article assembly web (113), the web of chassis material (154) having a machine direction (MD); and
cutting the article assembly web (113) to produce an individual article (20).

2. The method of claim 1, further comprising cutting the strip (118) after attaching the strip (118) to the web of chassis material (154).

3. The method of claim 2, wherein the cutting is in registry with the back leg band strip portion (148).

4. The method of claim 1, wherein the longitudinal direction is substantially parallel to the MD, and further comprising rotating the strip (118) such that the long dimension is substantially parallel to the MD.

5. The method of claim 1, wherein the back leg band stripe (144) is discontinuous.

6. The method of claim 5, wherein the back leg band stripe (144) includes an arcuate portion or wherein the back leg band stripe (144) includes a chevron portion.

7. The method of claim 5, wherein printing the back leg band stripe (144) includes printing part of the back leg band strip (144) at an angle to the waistband stripe (140); or wherein printing the back leg band stripe (144) includes printing part of the back leg band strip (144) to be parallel to the waistband stripe (140).

8. The method of claim 1, wherein the web of side panel material (116) is stretchable in the longitudinal direction; or wherein the web of side panel material (116) is stretchable in a direction perpendicular to the longitudinal direction.

9. The method of claim 1, wherein the web of chassis material (154) is printed with a back leg band segment.

10. The method of claim 1, wherein the front leg band stripe (142) and the waistband stripe (140) are parallel and spaced apart.

11. The method of claim 1, wherein the web of chassis material (154) includes outer cover material (40), liner material (42) generally parallel to the outer cover material (40), and absorbent core material (114) disposed therebetween.

12. The method of claim 1, wherein the waistband stripe (140) and the waistband stripe portion (149) have an unprinted stripe therein.

13. A method for manufacturing a disposable absorbent article (20) having multiple printed components, the method comprising:
printing a web of side panel material (116) with a front leg band stripe (142), a back leg band stripe (144), and a waistband stripe (140), the web of side panel material (116) having a longitudinal direction;
printing a web of chassis material (154) with a back leg band segment, the web of chassis material (154) having a machine direction (MD), outer cover material (40), liner material (42) generally parallel to the outer cover material (40), and absorbent core material (114) disposed therebetween, wherein the longitudinal direction is substantially parallel to the MD;
separating a strip (118) from the web of side panel material (116), the strip (118) having a long dimension and a front leg band stripe portion (142), a back leg band stripe portion (148), and a waistband stripe portion (140);
rotating the strip (118) such that the long dimension is substantially parallel to the MD;
attaching the strip (118) to the web of chassis material (154) to form an article assembly web (113); and
cutting the article assembly web (113) to produce an individual article (20).

14. The method of claim 1 or 13, wherein attaching the strip (118) to the web of chassis material (154) includes aligning the back leg band stripe portion (148) with the back leg band segment.

15. The method of claim 1 or 13, wherein cutting the article assembly web (113) includes cutting the strip (118) within the waistband stripe portion (149).

## Patentansprüche

1. Verfahren zur Herstellung eines saugfähigen Wegwerfartikels (20) mit mehreren aufgedruckten Komponenten, wobei das Verfahren umfasst:
Bereitstellen eines Gewebes aus Seitenwand (116) mit einer longitudinalen Richtung;
Separieren eines Streifens (118) aus dem Gewebe des Seitenwandmaterials (116), wobei der Streifen (118) eine lange Abmessung aufweist;
wobei das Verfahren Bedrucken des Streifens (118) oder des Gewebes (116) des Seitenwandmaterials mit einem vorderen Schenkelbandstreifen (142), einem hinteren Schenkelbandstreifen (144) und einem Hüftbandstreifen (140) vor dem Anbringen des Streifens (118) an dem Gewebe des Unterbaumaterials (154) umfasst, wobei der Streifen (118) einen vorderen Schenkelbandstreifenabschnitt (146), einen hinteren Schenkelbandstreifenschnitt (148) und einen Hüftbandstreifenabschnitt (149) aufweist;
Anbringen des Streifens (118) an ein Gewebe des Unterbaumaterials (154), um ein Artikel-Montagegewebe (113) zu bilden, wobei das Gewebe des Unterbaumaterials (154) eine Maschinenrichtung (MR) aufweist; und
Schneiden des Artikel-Montagegewebes (113), um einen individuellen Artikel (20) zu produzieren.

2. Verfahren nach Anspruch 1, ferner umfassend Schneiden des Streifens (118) nach dem Anbringen des Streifens (118) an das Gewebe des Unterbaumaterials (154).

3. Verfahren nach Anspruch 2, wobei das Schneiden in Registratur mit dem hinteren Schenkelbandstreifenabschnitt (148) erfolgt.

4. Verfahren nach Anspruch 1, wobei die longitudinale Richtung im Wesentlichen parallel zu der MR ist, und ferner umfassend Drehen des Streifens (118), so dass die lange Abmessung im Wesentlichen parallel ist zu der MR.

5. Verfahren nach Anspruch 1, wobei der hintere Schenkelbanstreifen (144) diskontinuierlich ist.

6. Verfahren nach Anspruch 5, wobei der hintere Schenkelbandstreifen (144) einen bogenförmigen Abschnitt beinhaltet, oder wobei der hintere Schenkelbandstreifen (144) einen Zickzackabschnitt beinhaltet.

7. Verfahren nach Anspruch 5, wobei Bedrucken des hinteren Schenkelbandstreifens (144) Bedrucken eines Teils des hinteren Schenkelbandstreifens (144) in einem Winkel zu dem Hüftbandstreifen (140) beinhaltet; oder wobei Bedrucken des hinteren Schenkelbandstreifens (144) Bedrucken eines Teils des hinteren Schenkelbandstreifens (144) beinhaltet, um parallel zu dem Hüftbandstreifen (140) zu sein.

8. Verfahren nach Anspruch 1, wobei das Gewebe des Seitenwandmaterials (116) in die longitudinale Richtung dehnbar ist; oder wobei das Gewebe des Seitenwandmaterials (116) in eine zu der longitudinalen Richtung rechtwinklige Richtung dehnbar ist.

9. Verfahren nach Anspruch 1, wobei das Gewebe des Unterbaumaterials (154) mit einem hinteren Schenkelbandsegment bedruckt ist.

10. Verfahren nach Anspruch 1, wobei der vordere Schenkelbandstreifen (142) und der Hüftbandstreifen (140) parallel und voneinander beabstandet sind.

11. Verfahren nach Anspruch 1, wobei das Gewebe des Unterbaumaterials (154) ein äußeres Deckmaterial (40), Futtermaterial (42), das im Allgemeinen parallel zu dem äußeren Deckmaterial (40) ist, und absorbierendes Kernmaterial (114) beinhaltet, das dazwischen angeordnet ist.

12. Verfahren nach Anspruch 1, wobei der Hüftbandstreifen (140) und der Hüftbandstreifenabschnitt (149) einen unbedruckten Streifen darin aufweisen.

13. Verfahren zu Herstellung eines absorbierenden Wegwerfartikels (20) mit mehreren aufgedruckten Komponenten, wobei das Verfahren umfasst:
Bedrucken eines Gewebes aus Seitenwandmaterial (116) mit einem vorderen Schenkelbandstreifen (142), eines hinteren Schenkelbandstreifens (144), und eines Hüftbandstreifens (140), wobei das Gewebe des Seitenwandmaterials (116) eine longitudinale Richtung aufweist;
Bedrucken eines Gewebes aus Unterbaumaterial (154) mit einem hinteren Schenkelbandsegment, des Gewebes aus Unterbaumaterial (154) mit einer Maschinenrichtung (MR), einem äußeren Abdeckmaterial (40), einem Futtermaterial (42), das im Allgemeinen parallel zu dem äußeren Abdeckmaterial (40) ist, und absorbierendem Kernmaterial (114), das dazwischen angeordnet ist, wobei die longitudinale Richtung im Wesentlichen parallel zu der MR ist;
Separieren eines Streifens (118), aus dem Gewebe des Seitenwandmaterials (116), wobei der Streifen (118) eine lange Abmessung aufweist und einen vorderen Schenkelbandstreifenabschnitt (142), eines hinteren Schenkelbandstreifenabschnitts (148), und eines Hüftbandstreifenabschnitts (140);
Rotieren des Streifens (118), derart, dass die lange Abmessung im Wesentlichen parallel zu der MR ist;
Anbringen des Streifens (118) an das Gewebe aus Unterbaumaterial (154), um ein Artikel-Montagegewebe (113) zu bilden; und
Schneiden des Artikel-Montagegewebes (113), um einen individuellen Artikel (20) herzustellen.

14. Verfahren nach Anspruch 1 oder 13, wobei Anbringen des Streifens (118) an das Gewebe des Unterbaumaterials (154) Anpassen des hinteren Schenkelbandstreifenabschnitts (148) mit dem hinteren Schenkelbandsegment beinhaltet.

15. Verfahren nach Anspruch 1 oder 13, wobei Schneiden des Artikel-Montagegewebes (113) Schneiden des Streifens (118) innerhalb des Hüftbandstreifenabschnitts (149) beinhaltet.

## Revendications

1. Procédé pour fabriquer un article absorbant jetable (20) ayant de multiples composants imprimés, le procédé comprenant :
la fourniture d'une toile de panneau latéral (116) ayant une direction longitudinale ;
la séparation d'une bande (118) de la toile de matériau de panneau latéral (116), dans lequel la bande (118) a une dimension longue ;
dans lequel le procédé comprend l'impression de la bande (118) ou de la toile (116) de matériau de panneau latéral avec une bandelette de jambe avant (142), une bandelette de jambe arrière (144) et une bandelette de taille (140) avant de fixer la bande (118) à la toile de matériau de châssis (154), dans lequel la bande (118) a une portion de bandelette de jambe avant (146), une portion de bandelette de jambe arrière (148) et une portion de bandelette de taille (149) ;
la fixation de la bande (118) à une toile de matériau de châssis (154) pour former une toile d'assemblage d'article (113), la toile de matériau de châssis (154) ayant une direction machine (MD) ; et
la découpe de la toile d'assemblage d'article (113) afin de produire un article individuel (20).

2. Procédé selon la revendication 1, comprenant en outre la découpe de la bande (118) après la fixation de la bande (118) à la toile de matériau de châssis (154).

3. Procédé selon la revendication 2, dans lequel la découpe est en alignement sur la portion de bandelette de jambe arrière (148).

4. Procédé selon la revendication 1, dans lequel la direction longitudinale est sensiblement parallèle à la MD, et comprenant en outre la rotation de la bande (118) de telle sorte que la dimension longue est sensiblement parallèle à la MD.

5. Procédé selon la revendication 1, dans lequel la bandelette de jambe arrière (144) est discontinue.

6. Procédé selon la revendication 5, dans lequel la bandelette de jambe arrière (144) inclut une portion arquée ou dans lequel la bandelette de jambe arrière (144) inclut une portion à chevrons.

7. Procédé selon la revendication 5, dans lequel l'impression de la bandelette de jambe arrière (144) inclut l'impression d'une partie de la bandelette de jambe arrière (144) selon un angle par rapport à la bandelette de taille (140) ; ou dans lequel l'impression de la bandelette de jambe arrière (144) inclut l'impression d'une partie de la bandelette de jambe arrière (144) pour qu'elle soit parallèle à la bandelette de taille (140).

8. Procédé selon la revendication 1, dans lequel la toile de matériau de panneau latéral (116) est étirable dans la direction longitudinale ; ou dans lequel la toile de matériau de panneau latéral (116) est étirable dans une direction perpendiculaire à la direction longitudinale.

9. Procédé selon la revendication 1, dans lequel la toile de matériau de châssis (154) est imprimée avec un segment de bande de jambe arrière.

10. Procédé selon la revendication 1, dans lequel la bandelette de jambe avant (142) et la bandelette de taille (140) sont parallèles et espacées.

11. Procédé selon la revendication 1, dans lequel la toile de matériau de châssis (154) inclut un matériau de protection extérieur (40), un matériau de revêtement (42) généralement parallèle au matériau de protection extérieur (40) et un matériau central absorbant (114) disposé entre eux.

12. Procédé selon la revendication 1, dans lequel la bandelette de taille (140) et la portion de bandelette de taille (149) ont une bandelette non imprimée dans celles-ci.

13. Procédé pour fabriquer un article absorbant jetable (20) ayant de multiples composants imprimés, le procédé comprenant :
l'impression d'une toile de matériau de panneau latéral (116) avec une bandelette de jambe avant (142), une bandelette de jambe arrière (144) et une bandelette de taille (140), la toile de matériau de panneau latéral (116) ayant une direction longitudinale ;
l'impression d'une toile de matériau de châssis (154) avec un segment de bande de jambe arrière, la toile de matériau de châssis (154) ayant une direction machine (MD), un matériau de protection extérieur (40), un matériau de revêtement (42) généralement parallèle au matériau de protection extérieur (40) et un matériau central absorbant (114) disposé entre eux, dans lequel la direction longitudinale est sensiblement parallèle à la MD ;
la séparation d'une bande (118) de la toile de matériau de panneau latéral (116), la bande (118) ayant une dimension longue et une portion de bandelette de jambe avant (142), une portion de bandelette de jambe arrière (148) et une portion de bandelette de taille (140) ;
la rotation de la bande (118) de telle sorte que la dimension longue est sensiblement parallèle à la MD ;
la fixation de la bande (118) à la toile de matériau de châssis (154) pour former une toile d'assemblage d'article (113) ; et
la découpe de la toile d'assemblage d'article (113) afin de produire un article individuel (20).

14. Procédé selon la revendication 1 ou 13, dans lequel la fixation de la bande (118) à la toile de matériau de châssis (154) inclut l'alignement de la portion de bandelette de jambe arrière (148) sur le segment de bande de jambe arrière.

15. Procédé selon la revendication 1 ou 13, dans lequel la découpe de la toile d'assemblage d'article (113) inclut la découpe de la bande (118) dans la portion de bandelette de taille (149).
